# EUROPÄISCHE PATENTSCHRIFT

(11) **EP 2 910 933 B1**
(45) Veröffentlichungstag und Bekanntmachung des Hinweises auf die Patenterteilung: **14.06.2017**
(21) Anmeldenummer: 14156620.8
(22) Anmeldetag: 25.02.2014
(51) Int. Cl.: G01N 21/82, G01N 33/28

(54) **Verfahren zur Ermittlung des Gesamteisengehalts in einer Probe eines flüssigen Schmieröls**
Method for determining the total iron content in a sample of a liquid lubricating oil
Procédé de détermination de la teneur totale en fer dans un échantillon d'une huile de lubrification liquide

(43) Veröffentlichungstag der Anmeldung: 26.08.2015
(73) Patentinhaber: CM Technologies GmbH, 35337 Elmshorn (DE)
(72) Erfinder: Jeske, Andreas, 25336 Elmshorn (DE); Winkler, Matthias, 25337 Elmshorn (DE)
(74) Vertreter: Meyer, Ludgerus

(56) Entgegenhaltungen:
- CN-A- 101 451 950
- CN-A- 101 975 688
- CN-A- 103 163 183
- US-A- 4 238 197
- US-A- 4 324 758
- US-A1- 2006 270 050
- SALVADOR A ET AL: "Determination of the total iron content of used lubricating oils by atomic-absorption with use of emulsions", TALANTA, ELSEVIER, AMSTERDAM, NL, Bd. 30, Nr. 12, 1. Dezember 1983 (1983-12-01), Seiten 986-988, XP026596345, ISSN: 0039-9140, DOI: 10.1016/0039-9140(83)80230-6 [gefunden am 1983-12-01]

## Beschreibung

Die Erfindung betrifft ein Verfahren zur Ermittlung des Gesamteisengehalts in einer Probe eines Schmieröls, insbesondere einer Schiffsmaschine.

Die in Handelsschiffen, insbesondere Containerschiffen, Tankern usw. verwendeten Schiffsmaschinen sind in der Regel langsam laufende Motoren mit fünf und mehr Zylindern, wobei die Motoren eine Höhe von mehr als 10 Metern erreichen können. Die gewünschte hohe Lebensdauer von über 20 Jahren erfordert es, dass der Betriebszustand solcher Motoren ständig überwacht wird.

Bei Großdieselmotoren für einen derartigen Einsatz erfolgt die Schmierung der Kolbenringe zu den Laufbuchsen in der Regel getrennt von der Schmierung des Kurbeltriebes. Dabei erfolgt die Schmierung zwischen den Kolbenringen und Laufbuchsen häufig als Verlustschmierung, wobei verbranntes Schmieröl bei jedem Kolbenhub durch den untersten Kolbenring abgestreift und entsorgt wird. Große Containerschiffe können bis zu einer Tonne Schmieröl pro Tag "verbrauchen". Es besteht daher das Bestreben, den Schmierölverbrauch möglichst weit zu reduzieren, ohne dass gleichzeitig die Gefahr vergrößert wird, eine Unterschmierung hervorzurufen, was zu großen Schäden am Motor führen kann.

Der Verschleiß eines Großdieselmotors tritt einerseits als mechanischer Abrieb auf, andererseits jedoch auch durch Säurekorrosion aufgrund bestimmter Kraftstoffeigenschaften, wie z. B. dem Schwefelgehalt. Korrosion tritt verstärkt dann auf, wenn ein Motor nicht bei optimalen Bedingungen betrieben wird, sondern der Motor beispielsweise bei langsamer Fahrt mit niedrigerer Temperatur arbeitet.

Im Schmieröl eines Motors finden sich daher nicht nur Abriebeisenpartikel, sondern z. B. auch korrodierte Eisenteilchen in Form von Eisenoxyd oder Eisensulfat. Um eine lange Lebensdauer des Motors zu gewährleisten, ist es notwendig, ständig den genauen Eisenanteil, sowohl des Abriebeisens als auch des korrodierten Eisens, zu überwachen.

Bei einer Verlustschmierung ist der Eisenanteil auch ein Gradmesser für den momentanen Verschleißzustand von Zylindern und Kolbenringen.

Zu Bestimmung des Alterungszustandes eines Schmieröls sind verschiedene Messverfahren bekannt, die unterschiedliche Eigenschaften des Schmieröls erfassen. Dazu gehören neben dem Eisengehalt die Säurezahl, der Additivgehalt, die Basenzahl, die Viskosität und die Leitfähigkeit.

Aus der DE 197 06 486 B4 ist eine Sensoreinrichtung zur Bestimmung des Alterungszustandes flüssiger Öle bekannt, welche zwei Interdigitalwandler zur Erzeugung einer elektroakustischen Welle verwendet, wobei zur Bewertung des Öls die ermittelte Resonanzfrequenz oder die Dämpfung der elektroakustischen Welle zwischen den Interdigitalwandlern ausgewertet wird, insbesondere indem die komplexe elektrische Impedanz mittels einer Auswertungsschaltung über eine Auswertung einer zweiten Resonanzfrequenz ermittelt wird.

Es ist auch bekannt, die magnetischen oder Leiteigenschaften einer Testflüssigkeit dadurch auszuwerten, dass magnetisierbare Partikel durch Änderung der Impedanz in einem elektrischen Feld gemessen werden, siehe US 6,051,970.

Das vorstehende Verfahren ist jedoch nur dafür geeignet, magnetisierbare Eisenteilchen in einem Öl festzustellen oder andere magnetfeldabhängige Parameter zu ermitteln.

US 2006/270050 A1 offenbart ein Verfahren zur Ermittlung des Eisengehalts in einer Probe eines flüssigen Schmiermittels mittels sichtbarer Spektroskopie wobei das zu bestimmende Schmiermittel ein Öl ist, das zum Schmieren von Maschinen oder Motoren verwendet wird. Das Verfahren umfasst die Zugabe einer vorbestimmten Menge des Schmiermittels zu einem aktiven Lösungsmittel, umfassend eine vorbestimmte Menge eines apolaren organischen Lösungsmittels, eines polaren organischen Lösungsmittels, einer organischen Säure, Wasser, eines Eisenkomplexbildners und einem Reduktionsmittel, wobei das aktive Lösungsmittel einen pH zwischen 2 und 4 aufweist. Weiterhin werden das Schmiermittel und das aktive Lösungsmittel gründlich vermischt, bis das Schmiermittel vollständig gelöst ist. Das Gemisch lässt man vollständig reagieren bis es sich in eine obere Schicht und eine unteren Schicht trennt, wobei die untere Schicht einen Eisen-Komplex umfasst. Nach Filtern wenigstens
ein Teil der unteren Schicht aus der Mischung direkt in ein, für eine Absorptionsmessung,
im sichtbaren Bereich, geeignetes Gefäß wird die Nettoabsorption der filtrierten Lösung
bei Frequenzen im sichtbaren Bereich photometrische gemessen. Ein Umwandeln der Absorptionsmessung ergibt den Eisengehalt des Schmiermittels in ppm. Es ist Aufgabe der Erfindung, ein Verfahren zur Ermittlung des Gesamteisengehalts eines Schmieröls anzugeben, mit dem der momentane Verschleißzustand eines Motors und insbesondere dessen Änderung ermittelt werden kann.

Diese Aufgabe wird durch die in Anspruch 1 angegebene Erfindung gelöst. Vorteilhafte Weiterbildungen der Erfindung sind in den Unteransprüchen angegeben.

Das offenbarte Verfahren zur Ermittlung des Gesamteisengehaltes in einer Probe eines flüssigen Schmieröls umfasst die Schritte zunächst eine Probe eines Schmieröls in einem Mischbehälter mit einer Eisenionen bildenden Säure zu vermischen. Der Mischbehälter wird dann in ein Ultraschallbad eingebracht. Bei erhöhter Temperatur wird die Mischung mindestens so lange dem Ultraschallfeld unterzogen, bis eine Phasentrennung in der Mischung in zwei Phasen mit unterschiedlichem spezifischem Gewicht erfolgt, wobei die schwerere Phase, welche Eisenionen enthält, und die leichtere Phase, welche Ölreste enthält, durch eine Phasentrennebene voneinander getrennt sind. Die schwerere Phase, d.h. die Flüssigkeitsphase, welche ein höheres spezifisches Gewicht aufweist, wird dann durch einen engmaschigen Filter aus dem Mischbehälter in ein Reaktionsgefäß überführt, in dem sich eine Lösung eines Eisenkomplexbildners befindet. Die dabei entstehende Trübung oder Färbung der Flüssigkeit (Chrominanz oder Luminanz) lässt sich auswerten und ergibt ein Maß für den Gesamteisengehalt in der Probe.

Es wird bevorzugt, als Eisenionen bildende Säure verdünnte Salpetersäure zu verwenden, insbesondere, weil diese in der Handhabung insbesondere im rauen Schiffsbetrieb relativ ungefährlich ist, obgleich auch andere Säuren, insbesondere Salzsäure, verwendbar sind. Vorzugsweise wird die Eisenionen bildende Säure, die als Salpetersäure eine 10 - 50%ige Lösung in H₂O sein kann, zunächst in einen Mischbehälter eingegeben, dem dann die Probe in einem 0.1 : 1 bis 10 : 1 Verhältnis von Probe zu Säure zugeführt wird. Vorzugsweise werden gleiche Volumina von Probe und Säure verwendet.

Der Mischbehälter ist vorzugsweise ein zylindrisches Röhrchen, in die die Eisenionen bildende Säure und die Probe eingebracht werden, wobei zur Erleichterung der Mischung ein Teilvolumenraum des Mischbehälters mit Luft gefüllt bleibt.

Das Einbringen des Mischbehälters in einen Ulltraschallerzeuger erfolgt bei einem zylindrischen Behälter mit gegenüber dem Durchmesser großer Höhe in geneigter Stellung des Mischbehälters, derart, dass die sich bei der Ultraschallbehandlung ergebende Phasentrennung eine möglichst große Trennebene hervorruft. Dieses erleichtert sowohl die Durchmischung der Eisenionen bildenden Säure mit der Ölprobe als auch die anschließende Ausbildung der Phasentrennebene.

Alternativ wird ein Mischbehälter verwendet werden, dessen Höhe kleiner als der Durchmesser gewählt ist, wodurch keine Neigung des Mischbehälters erforderlich ist. Als Mischbehälter sollte ein Behälter mit harter Oberfläche benutzt werden. Vorzugsweise wird ein Mischbehälter aus Glas verwendet.

Im Ultraschallbad erfolgt die Behandlung mit Ultraschall mit einer Leistung von > 20 W, einer Ultraschallfrequenz von > 30 kHz und einer Zeitdauer von wenigstens 5 min bei einer Temperatur des Ultraschallbades von > 40°C. In einem bevorzugten Ausführungsbeispiel wird eine Leistung des Ultraschallerzeugers von 30 bis 60 W, eine Ultraschallfrequenz von 35 bis 45 kHz und eine Zeitdauer von 10 bis 60 min bei einer Temperatur des Ultraschallbades von 40 - 80°C gewählt. Diese Parameter können in der praktischen Umsetzung anhand der erzielten Ergebnisse weiter optimiert werden.

Durch den Einsatz von Ultraschall werden die Flüssigkeiten im Mischbehälter, nämlich die Ölphase mit abrasiven und korrosiven Eisenbestandteilen und die wässrige HNO3-Phase, in kleinste Schwingungen versetzt, wodurch sich folgende Vorteile ergeben:
a) Für korrosives Eisen: Durch die anfängliche Zufuhr mechanischer Energie (Schütteln) werden temporär zwei Phasen (HNO₃ und Öl) die sich unter Normalbedingungen nicht stabil durchmengen lassen, vermischt. Dadurch wird erreicht, dass möglichst viel Säure mit dem im Öl vorhandenen Eisen in Kontakt kommt. Danach wird der Mischbehälter in das Ultraschallbad eingebracht. Hier wird die natürliche Trennung (Entmischung) der beiden Phasen durch Einwirkung von Temperatur und Ultraschallenergie beschleunigt. Danach erfolgt durch die erhöhte Temperatur und die Ultraschallenergie eine Konvektion, die ständig eine Veränderung der Molekülzusammensetzung an der Phasentrennebene bewirkt. Dieser Prozess fördert den immer weiteren Übertrag von ionisiertem Eisen in die wässrige HNO₃-Phase.
b) Für abrasives Eisen: Kleinste abrasive Eisenteilchen im zweistelligen Nanometerbereich sinken in Stoffen wie Ölen mit hoher Viskosität nicht zeitnah selbsttätig ab, trotz höherer Dichte der Eisenteilchen gegenüber dem Öl, wie das in dünneren Medien, wie zum Beispiel Wasser, oder bei größeren Teilchen der Fall wäre. Mittels Ultraschall werden diese nun "herausgeschüttelt" und "wandern" in die untere Phase. Die Temperatur erhöht die Brownsche Molekularbewegung der Teilchen, die in diesem Zusammenhang die Viskosität der Ölphase herabsetzt, wodurch dieser Effekt weiter unterstützt wird.

Nach der Ultraschallbadbehandlung wird die die Eisenionen enthaltende schwerere Phase der Mischung abfiltriert. Das Filtrat wird in ein Reaktionsgefäß überführt, in dem sich ein Eisenkomplexbildner befindet. Dieser kann sowohl ein Eisen-II- als auch ein Eisen-III-Komplexbildner sein. Als Eisen-II-Komplexbildner wird insbesondere Kaliumhexazyanidoferrat-III und als Eisen-III-Komplexbildner Kaliumhexazyanodidoferrat-II verwendet. Kaliumhexazyanidoferrat-II ist auch als gelbes Blutlaugensalz bekannt, während Kaliumhexazyanodidoferrat-III als rotes Blutlaugensalz bekannt ist, welche beide häufig zur Herstellung von Blaupigmenten und Blaudrucken verwendet werden. Bei der Vermischung des gelben bzw. roten Blutlaugensalzes mit Eisenionen entsteht das sogenannte Berliner Blau, das sich durch Blaufärbung der erhaltenen Lösung kennzeichnet. Der Grad der Verfärbung in Form einer Trübung, der Änderung der Chominanz oder der Luminanz ist ein Maß für die ermittelte Eisenionenmenge.

Die Auswertung kann durch Augenschein vorgenommen werden, jedoch wird es bevorzugt, die Auswertung durch ein Trübungs- oder Färbungsmessgerät bekannter Art vorzunehmen, um reproduzierbare Ergebnisse dokumentieren zu können.

Mit dem vorstehenden Verfahren ist es möglich, den Gesamteisengehalt in einer Probe eines Öls, insbesondere Schmieröls einer Schiffsmaschine, zu bestimmen, und damit den Verschleißzustand des Öls und/oder des Motors ermitteln zu können. Die erfindungsgemäßen Verfahrensschritte können unmittelbar an Bord eines Schiffes durchgeführt werden, ohne dass dafür eine komplexe Labortechnik vorhanden sein muss.

Die Erfindung wird nachstehend anhand eines Beispiels näher erläutert.

In einem Beispiel wurde als Probenbehälter ein 10 ml Glasspritzenbehälter mit einem Druck- oder Zugkolben verwendet, in den durch Aufziehen des Kolbens 4 ml einer 0,1-molaren Lösung von Salpetersäure in H₂O aufgezogen wurde. Es wurden ferner eine repräsentative Probe von 4 ml Schmieröl aus einer Schiffsmaschine sowie weitere 2 ml Luft in die Spritze aufgezogen. Es folgte eine initiale Vermischung der Salpetersäure mit der Probe durch händisches Schütteln über 10 sec oder etwa 30-maliges Hin- und Herschütteln. Dann wurde die Spritze in ein Ultraschallbad eingebracht, das eine Leistung von 50 W bei einer Ultraschallfrequenz von 42 kHz aufwies. Die Temperatur des Ultraschallbades betrug 60°C. Der Mischbehälter wurde für 30 min in dem Ultraschallbad behandelt, wobei der Mischbehälter (die Spritze) schräg im Ultraschallbad gelagert wurde, so dass in dem Mischbehälter eine möglichst große Fläche der Phasentrennebene zwischen der leichteren und der schwereren Phase erreicht werden konnte. Nach Beendigung der Ultraschallbehandlung wurde die Spritze aufrecht aus dem Ultraschallbad entnommen und an einen Filter mit einer 0,2 µm Porengröße angeschlossen. Das Filtrat wurde in ein Gewindeglas mit 30 ml Volumen überführt, in das zuvor eine 10 ml Lösung aus gelbem Blutlaugensalz in 50 mg/l gefiltertem Wasser eingeführt wurde. Nach einer Wartezeit von 15 min erfolgte eine weitere Verdünnung mit 15 ml gefiltertem Wasser. Das Gewindeglas wurde dann in die Messzelle eines fotoelektrischen Messgeräts eingebracht, um die Trübung bzw. Färbung der erhaltenen Lösung festzustellen. Durchgeführte Messreihen mit einem Frischöl, einem stark mit Eisen versetztem Schmieröl und Ölen mit verschiedenen Abstufungen des Gesamteisengehaltes führten zu wiederholbaren Ergebnissen, die ein Maß für den Gesamteisengehalt einer Probe ergaben.

In einem weiteren Versuch wurde festgestellt, dass die Verweildauer der Probenmischung im Ultraschallbad 10 min nicht unterschreiten sollte, da bei geringerer Zeitdauer keine ausreichende Trennung zwischen der Eisenionen enthaltenden Phase und Ölresten erhältlich war. Grundsätzlich war es von Vorteil, die Temperatur der Ultraschallbehandlung zu erhöhen, jedoch ohne eine Temperatur von 80°C zu überschreiten.

## Patentansprüche

1. Verfahren zur Ermittlung des Gesamteisengehalts in einer Probe eines flüssigen Schmieröls, insbesondere einer Schiffsmaschine, mittels folgender Schritte:
a) Aus dem Schmieröl wird eine Probe entnommen,
b) Die Probe und eisenionenbildende verdünnte Salpetersäure (HNO₃) werden einem Mischbehälter zugeführt und vermischt,
c) Der Mischbehälter wird in ein Ultraschallbad eingebracht und bei erhöhter Temperatur wenigstens solange einem Ultraschallfeld unterzogen, bis eine Phasentrennung in der Mischung mit einer schwereren Phase, welche Eisenionen enthält, und einer leichteren Phase, welche Ölreste enthält, erfolgt ist, - wobei der Mischbehälter entweder ein Höhe-zu-Durchmesser-Verhältnis von < 1 hat oder derart geneigt wird, dass die Fläche der Phasentrennebene möglichst groß ist,
d) Die schwerere Phase der Mischung wird ausfiltriert, und die das Filtrat bildende schwerere Phase wird in ein Reaktionsgefäß überführt, welches eine Lösung eines Eisenkomplexbildners enthält,
e) Der Eisengehalt der Probe wird über die Feststellung der Trübung, der Chrominanz oder der Luminanz einer Farbbildung in dem Reaktionsgefäß ausgewertet.

2. Verfahren nach Anspruch 1, **dadurch gekennzeichnet, dass** die Probe und die Salpetersäure in einem Verhältnis von 0,1 : 1 bis 10 : 1 Volumenanteile dem Mischbehälter zugeführt werden.

3. Verfahren nach Anspruch 2, **dadurch gekennzeichnet, dass** die Volumina von Probe und Salpetersäure im Wesentlichen gleich sind.

4. Verfahren nach Anspruch 3, **dadurch gekennzeichnet, dass** die Volumina jeweils 1 - 40 ml betragen.

5. Verfahren nach Anspruch 1, **dadurch gekennzeichnet, dass** die Salpetersäure eine 10 - 50%ige Lösung in H₂O ist.

6. Verfahren nach Anspruch 1 oder 5, **dadurch gekennzeichnet, dass** die Salpetersäure steril gefiltert und mit hochreinem Wasser verdünnt wird.

7. Verfahren nach Anspruch 1, **dadurch gekennzeichnet, dass** die Behandlung im Ultraschallbad mit einem Ultraschallerzeuger mit einer Leistung von > 20 Watt, einer Ultraschallfrequenz von > 30 kHz und einer Zeitdauer von wenigstens 5 min bei einer Temperatur des Ultraschallbades von > 40 °C erfolgt.

8. Verfahren nach Anspruch 8, **dadurch gekennzeichnet, dass** die Behandlung im Ultraschallbad mit einem Ultraschallerzeuger mit einer Leistung von 30 - 60 Watt, einer Ultraschallfrequenz von 35 - 45 kHz und einer Zeitdauer von 10 - 60 min bei einer Temperatur des Ultraschallbades von 40 - 80 °C erfolgt.

9. Verfahren nach Anspruch 1, **dadurch gekennzeichnet, dass** der Eisenkomplexbildner ein Eisen-II- oder Eisen-III-Komplexbildner ist.

10. Verfahren nach Anspruch 10, **dadurch gekennzeichnet, dass** der Eisen-III Komplexbilder Kaliumhexacyanidoferrat (II) K₄[Fe(CN)₆] ist.

11. Verfahren nach Anspruch 10, **dadurch gekennzeichnet, dass** der Eisen-II-Komplexbilder Kaliumhexacyanidoferrat (III) K₃[Fe(CN)₆] ist.

## Claims

1. Method for determining the total iron content in a sample of a liquid lubricating oil, in particular from a marine engine, by means of the following steps:
a) a sample of the lubricating oil is taken,
b) the sample and diluted nitric acid (HNO₃) that forms iron ions are added to a mixing vessel and mixed,
c) the mixing vessel is placed in an ultrasonic bath and, at an increased temperature, is subjected to an ultrasonic field at least until a phase separation occurs in the mixture between a heavier phase containing iron ions and a lighter phase containing oil residues, the mixing vessel having a height to diameter ratio of < 1 or being inclined such that the surface area of the phase separation plane is as large as possible,
d) the heavier phase of the mixture is filtered out and the heavier phase forming the filtrate is transferred to a reaction vessel containing an iron complexing agent solution,
e) the iron content of the sample is analysed by determining the opacity, chrominance or luminance of a colour formation in the reaction vessel.

2. Method according to claim 1, **characterised in that** the sample and the nitric acid are added to the mixing vessel in a ratio of from 0.1:1 to 10:1 parts by volume.

3. Method according to claim 2, **characterised in that** the volumes of the sample and the nitric acid are substantially the same.

4. Method according to claim 3, **characterised in that** the volumes are 1-40 ml in each case.

5. Method according to claim 1, **characterised in that** the nitric acid is a 10-50 % solution in H₂O.

6. Method according to either claim 1 or claim 5, **characterised in that** the nitric acid is sterile filtered and diluted using high-purity water.

7. Method according to claim 1, **characterised in that** the treatment in the ultrasonic bath takes place using an ultrasonic generator having an output of > 20 watts, an ultrasonic frequency of > 30 kHz and a duration of at least 5 minutes at a temperature of the ultrasonic bath of > 40 °C.

8. Method according to claim 8, **characterised in that** the treatment in the ultrasonic bath takes place using an ultrasonic generator having an output of 30-60 watts, an ultrasonic frequency of 35-45 kHz and a duration of 10-60 minutes at a temperature of the ultrasonic bath of 40-80 °C.

9. Method according to claim 1, **characterised in that** the iron complexing agent is an iron(II) complexing agent or an iron(III) complexing agent.

10. Method according to claim 10, **characterised in that** the iron(III) complexing agent is potassium ferrocyanide K₄[Fe(CN)₆].

11. Method according to claim 10, **characterised in that** the iron(II) complexing agent is potassium ferricyanide K₃[Fe(CN)₆].

## Revendications

1. Procédé pour déterminer la teneur totale en fer dans un échantillon d'une huile de lubrification liquide, en particulier d'une machine de bateau, à l'aide des étapes suivantes :
a) un échantillon est prélevé de l'huile de lubrification,
b) l'échantillon et de l'acide nitrique (HNO₃) dilué formant des ions de fer sont amenés dans un récipient de mélange et sont mélangés,
c) le récipient de mélange est placé dans un bain à ultrasons et exposé à un champ d'ultrasons, à une température accrue, au moins jusqu'à ce qu'une séparation de phases ait eu lieu dans le mélange, avec une phase plus lourde qui contient des ions de fer et une phase plus légère qui contient des restes d'huile, le récipient de mélange présentant un rapport hauteur-diamètre < 1 ou étant incliné de telle sorte que la surface du plan de séparation de phases soit aussi grande que possible,
d) la phase plus lourde du mélange est filtrée et la phase plus lourde qui forme le filtrat est transférée dans un récipient de réaction qui contient une solution d'un agent de complexation de fer,
e) la teneur en fer de l'échantillon est évaluée par l'intermédiaire de la constatation du trouble, de la chrominance ou de la luminance d'une formation de couleur dans le récipient à réaction.

2. Procédé selon la revendication 1, **caractérisé en ce que** l'échantillon et l'acide nitrique sont amenés dans le récipient de mélange avec un rapport de 0,1:1 à 10:1 en volume.

3. Procédé selon la revendication 2, **caractérisé en ce que** les volumes de l'échantillon et de l'acide nitrique sont globalement égaux.

4. Procédé selon la revendication 3, **caractérisé en ce que** les volumes sont de 1-40 ml.

5. Procédé selon la revendication 1, **caractérisé en ce que** l'acide nitrique est une solution à 10-50 % dans H₂O.

6. Procédé selon la revendication 1 ou 5, **caractérisé en ce que** l'acide nitrique est filtré stérilement et est dilué avec de l'eau ultra-pure.

7. Procédé selon la revendication 1, **caractérisé en ce que** le traitement dans le bain à ultrasons a lieu avec un générateur d'ultrasons avec une puissance > 20 watts, une fréquence d'ultrasons > 30 kHz et une durée d'au moins 5 min. à une température de bain à ultrasons > 40°C.

8. Procédé selon la revendication 8, **caractérisé en ce que** le traitement dans le bain à ultrasons a lieu avec un générateur d'ultrasons avec une puissance de 30-60 watts, une fréquence d'ultrasons de 35-45 kHz et une durée de 10-60 min. à une température de bain à ultrasons de 40-80°C.

9. Procédé selon la revendication 1, **caractérisé en ce que** l'agent de complexation de fer est un agent de complexation de fer-II ou de fer-III.

10. Procédé selon la revendication 10, **caractérisé en ce que** l'agent de complexation de fer-III est de l'hexacyanoferrate de potassium (II) K₄[Fe(CN)₆].

11. Procédé selon la revendication 10, **caractérisé en ce que** l'agent de complexation de fer-II est de l'hexacyanoferrate de potassium (III) K₃[Fe(CN)₆].
